# EUROPÄISCHE PATENTSCHRIFT

(11) **EP 3 761 946 B1**
(45) Veröffentlichungstag und Bekanntmachung des Hinweises auf die Patenterteilung: **30.03.2022**
(21) Anmeldenummer: 19703271.7
(22) Anmeldetag: 30.01.2019
(51) Int. Cl.: A61K 8/365, A61K 8/368, A61K 8/67, A61Q 19/00, A61K 8/81, A61K 8/02, A61K 8/04

(54) **FRUCHTSÄURE-HALTIGES, KOSMETISCHES GEL**
FRUIT ACID-CONTAINING COSMETIC GEL
GEL COSMÉTIQUE CONTENANT DE L'ACIDE DE FRUIT

(30) Priorität: 05.03.2018 DE 102018203223
(43) Veröffentlichungstag der Anmeldung: 13.01.2021
(73) Patentinhaber: Beiersdorf AG, 20253 Hamburg (DE)
(72) Erfinder: DUNCKEL, GEB. RUHS, Joanna, 21649 Regesbostel (DE); HARBIG, Sabrina, 22529 Hamburg (DE); BLAUDOW, Luisa, 22043 Hamburg (DE); DJAMIL, Jane, 20357 Hamburg (DE)
(74) Vertreter: Beiersdorf AG
(86) Internationale Anmeldenummer: PCT/EP2019/052201
(87) Internationale Veröffentlichungsnummer: WO 2019/170327

(56) Entgegenhaltungen:
- EP-A1- 1 391 195
- EP-A1- 1 396 262
- EP-A1- 1 516 613
- WO-A1-01/64166
- WO-A1-2019/025115
- CH-A2- 711 092
- US-A1- 2004 253 313

## Beschreibung

Die Erfindung betrifft ein Kosmetikum gebildet aus einem Vorratsbehältnis mit zwei separaten Vorratskammern, enthaltend zwei unterschiedliche kosmetische Teilzuberei-tungen, die über eine gemeinsame Entnahmeöffnung gleichzeitig entnommen werden, wobei die erste Kammer ein wässrig oder wässrig-alkoholisches Gel und die zweite Kammer eine Säure-haltige Zubereitung mit einem pH-Wert unter pH 4 enthält.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren haben dabei der Zustand und das Aussehen der Haut.

Hautpflegeprodukte bestehen in der Regel aus Emulsionen. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-ÖI-Emulsion (W/O-Emulsion, z. B. Butter) handelt es sich um das umgekehrte Prinzip, wobei der Grundcharakter hier durch das Öl bestimmt wird.

Die Vielzahl an kommerziell erhältlichen kosmetischen Emulsionen darf jedoch nicht darüber hinwegtäuschen, dass diese Zubereitungen des Standes der Technik eine Reihe von Nachteilen aufweisen.

Um O/W-Emulsionen eine gewisse Konsistenz (Viskosität) und Stabilität zu geben und sie nicht zu dünnflüssig werden zu lassen, werden diesen Zubereitungen regelmäßig Gelbildner zugesetzt, die in der äußeren, wässrigen Phase ein Gelgerüst ausbilden. Die wässrige Phase wird damit zähflüssiger und das Zusammenfließen der Öltröpfchen wird unterdrückt. Neben Cellulose-Derivaten, die nur eine untergeordnete Rolle spielen, werden hierfür üblicherweise Polyacrylate eingesetzt. Polyacrylate haben den Vorteil, auch bei hohen Wassergehalten der Emulsion eine Phasentrennung zu unterbinden.

Nachteilig am Stande der Technik ist jedoch der Umstand, dass für die Ausbildung von Polyacrylat-Gelnetzwerken ein hinreichend hoher pH-Wert (größer pH 5) erforderlich ist, denn nicht die Acrylsäure selbst bildet das Netzwerk sondern nur ihre Salze. Aus diesem Grunde wird bei der Herstellung von Polyacrylat-Gelen zunächst die Polyacrylsäure in die Zubereitung eingearbeitet und anschließend mit einer Base (meist NaOH) neutralisiert. Erst bei der Neutralisation bildet sich dann das Gelnetzwerk.

Daher ist es nach dem Stande der Technik nicht möglich, saure Wirkstoffe, beispielweise Fruchtsäuren wie Glycolsäure, Milchsäure, Ascorbinsäure oder Zitronensäure in Polyacrylathaltige O/W-Emulsionen als Säuren einzuarbeiten.

Fruchtsäuren wie Glycolsäure, Milchsäure, Ascorbinsäure oder Zitronensäure werden in der Kosmetik nicht allein als kosmetisch wirksame Stoffe eingesetzt, wo die freien Säuren eine besondere Wirksamkeit entfalten. Durch ihre Säurewirkung wirken sie darüber hinaus auch antimikrobiell im Hinblick auf die Konservierung der Zubereitung selbst. Da es ein weit verbreiteter Wunsch der Verbraucher ist, möglichst "Konservierungsmittel-freie" (oder "Konservierungsmittel-reduzierte") Kosmetika zu verwenden, gibt es für die Konservierung mittels pH-Wert Erniedrigung ein wachsendes Interesse.

WO0164166 A1 offenbart ein Kosmetikum gebildet aus einem Vorratsbehältnis mit zwei separaten Vorratskammern, enthaltend zwei unterschiedliche kosmetische Teilzubereitungen, die über eine gemeinsame Entnahmeöffnung gleichzeitig entnommen werden, wobei die erste Kammer ein Wirkstoffhaltige Zusammensetzung und die zweite Kammer eine Säure-haltige Zubereitung mit einem pH-Wert unter pH 4 enthält (z.B. enthaltend eine Hydoxycarbonsäure). Die erste Zusammensetzung wird separat von der Säure-haltige Zubereitung enthalten wegen Inkompatibilität.

EP1391195 A1 offenbart ein Kosmetikum gebildet aus einem Vorratsbehältnis mit zwei separaten Vorratskammern enthaltend zwei unterschiedlichen Kosmetische Teilzubereitungen, die über eine gemeinsame Entnahmeöffnung gleichzeitig entnommen werde, wobei die erste Kammer eine Reinigungszubereitung enthält, und die zweite Kammer eine gelförmige Zubereitung enthält.

Es war daher die Aufgabe der vorliegenden Erfindung, eine "Konservierungsmittel-reduzierte", stabile, höher-viskose O/W-Emulsion zu entwickeln.

Überraschend gelöst wird die Aufgabe durch ein Kosmetikum, dass gebildet wird aus einem Vorratsbehältnis mit zwei separaten Vorratskammern, enthaltend zwei unterschiedliche kosmetische Teilzubereitungen, die über eine gemeinsame Entnahmeöffnung gleichzeitig entnommen werden, wobei die erste Kammer ein wässrig oder wässrig-alkoholisches Gel und die zweite Kammer eine Säure-haltige Zubereitung mit einem pH-Wert unter pH 4 enthält.

Dabei ist es erfindungsgemäß von Vorteil, wenn das Gel in der ersten Kammer durch Polyacrylat (Carbomer) verdickt ist. Erfindungsgemäß bevorzugt sind quervernetzte Polyacrylate, insbesondere solche mit hohem Vernetzungsgrad wie Carbopol 2984 von Lubrizol (CAS-Nummer: 9007-20-9, 9003-01-4, 76050-42-5, 9062-04-8, 9007-16-3, 9007-17-4).

Vorteilhafte Ausführungsformen der vorliegenden Erfindung sind dabei dadurch gekennzeichnet, dass das Gel in der ersten Kammer Polyacrylate in einer Konzentration von 0,1 bis 1,5 Gewichts-%, bezogen auf das Gesamtgewicht des Gels in der ersten Kammer, enthält.

Die erfindungsgemäß bevorzugte Einsatzkonzentration für die Polyacrylate in der ersten Kammer beträgt dabei Konzentration von 0,7 bis 1,3 Gewichts-%, bezogen auf das Gesamtgewicht des Gels in der ersten Kammer.

Nun mag es bei rückschauender Betrachtungsweise naheliegend erscheinen, Gel und Emulsion räumlich voneinander zu trennen und erst bei der Anwendung zusammenzufügen. Der Fachmann hatte diese Lösung jedoch nicht in Betracht gezogen, da er zunächst davon ausgehen musste, dass das Vermischen beider relativ hochviskosen Teilzubereitungen bei der Anwendung auf der Haut zu Komplikationen führen würde. Es war damit zu rechnen, dass sich die Teilzubereitungen nicht zu einer homogenen Gesamtzubereitung vermischen lassen. Ferner war damit zu rechnen, dass das Polyacrylat-Gelnetzwerk bei Kontakt mit der sauren Emulsion sofort wieder zusammenbricht. Dies ist bei den erfindungsgemäßen Zubereitungen so jedoch nicht der Fall. Nicht zuletzt war eine verstärkte "Röllchenbildung" beim Verreiben der beiden Teilzubereitungen zu befürchten, die bei den Verbrauchern unerwünscht ist und die kosmetische Wirksamkeit der Gesamtzubereitung reduziert.

Nicht zuletzt war bei der Variante des wässrig-alkoholischen Gels davon auszugehen, dass der olfaktorische Gesamteindruck des Produktes bei der Anwendung durch den Ethanol-Geruch dominiert wird. Im erfindungsgemäßen Produkt jedoch wird der Geruch durch die Parfümöle der Emulsion bestimmt.

Es ist erfindungsgemäß vorteilhaft, wenn der pH-Wert des wässrigen oder wässrig-alkoholischen Gels in der ersten Kammer größer als pH 4,5 ist, wobei ein Bereich von 4,5-5,5 erfindungsgemäß bevorzugt wird. Erfindungsgemäß besonders bevorzugt ist der Bereich von pH 4,9-5,2.

Es ist erfindungsgemäß vorteilhaft, wenn der pH-Wert der Zubereitung in der zweiten Kammer zwischen 3,0 und 4,0 liegt, wobei der pH-Bereich zwischen 3,5 und 3,7 erfindungsgemäß bevorzugt ist.

Es ist erfindungsgemäß von Vorteil, wenn das Gel in der ersten Kammer ein oder mehrere kosmetische Wirkstoffe gewählt aus der Gruppe der Verbindungen D-Biotin, Coenzym Q10, alpha-Glucosylrutin, Carnitin, Carnosin, natürliche und/oder synthetische Isoflavonoide, Flavonoide, Kreatin, Kreatinin, Taurin, ß-Alanin, Panthenol, Magnolol, Honokiol, Polydocanol, Glycerylglycose, Tocopherolacetat, Natriumhyaluronat, Harnstoff und/oder Licochalcon A enthält.

Dabei ist der Einsatz von Natriumhyaluronat erfindungsgemäß bevorzugt, welches erfindungsgemäß vorteilhaft in einer Konzentration von 0,1 bis 1 Gewichts-%, bezogen auf das Gesamtgewicht des Gels in der ersten Kammer, eingesetzt wird.

Darüber hinaus kann Natriumhyaluronat zusätzlich in die Säure-haltige Zubereitung der zweiten Kammer eingearbeitet werden.

Vorteilhafte Ausführungsformen der vorliegenden Erfindung sind dadurch gekennzeichnet, dass das Gel in der ersten Kammer Phenoxyethanol, Ethylhexylglycerin und/oder 4-Hydroxyacetophenon enthält. Dabei ist der Einsatz von Phenoxyethanol erfindungsgemäß bevorzugt. Die bevorzugte Einsatzkonzentration für Phenoxyethanol beträgt von 0,1 bis 1 Gewichts-%, bezogen auf das Gesamtgewicht des Gels in der ersten Kammer.

Außerdem ist es erfindungsgemäß von Vorteil, wenn das Gel in der ersten Kammer ein wässrig-alkoholisches Gel darstellt mit einem Ethanol-Gehalt von mindestens 4 Gewichts-% Ethanol, bezogen auf das Gesamtgewicht des Gels in der ersten Kammer. Ein Ethanol-Gehalt von mindestens 8 Gewichts-% Ethanol, bezogen auf das Gesamtgewicht des Gels in der ersten Kammer, ist erfindungsgemäß bevorzugt.

Es ist erfindungsgemäß vorteilhaft, wenn die Zubereitung in der zweiten Kammer ein oder mehrere Säuren, gewählt aus der Gruppe der Verbindungen Gluconsäure, Milchsäure, Ascorbinsäure, Zitronensäure, Salizylsäure, Glykolsäure enthält. Dabei kann die Gluconsäure erfindungsgemäß vorteilhaft auch ganz oder teilweise in Form ihres Lactons Gluconolacton eingesetzt werden. In einem solchen Falle werden bevorzugt Mischungen aus Gluconsäure und Gluconolacton eingesetzt.

Insbesondere haben sich Mischungen aus Glykolsäure, Gluconolacton und Milchsäure als erfindungsgemäß besonders wertvoll erwiesen.

Die erfindungsgemäß vorteilhafte Einsatzkonzentration für Glykolsäure beträgt von 0,1 bis 7 Gewichts-%, bezogen auf das Gesamtgewicht der Zubereitung in der zweiten Kammer.

Die erfindungsgemäß vorteilhafte Einsatzkonzentration für Gluconolacton beträgt von 0,1 bis 2 Gewichts-%, bezogen auf das Gesamtgewicht der Zubereitung in der zweiten Kammer.

Die erfindungsgemäß vorteilhafte Einsatzkonzentration für Milchsäure beträgt von 0,1 bis 3 Gewichts-%, bezogen auf das Gesamtgewicht der Zubereitung in der zweiten Kammer.

Erfindungsgemäß vorteilhafte Ausführungsformen der vorliegenden Erfindung sind dadurch gekennzeichnet, dass die Zubereitung in der zweiten Kammer in Form einer Öl-in-Wasser-Emulsion (O/W-Emulsion) vorliegt.

Erfindungsgemäß vorteilhaft ist es ferner, wenn die erfindungsgemäße Zubereitung in der zweiten Kammer ein oder mehrere Verbindungen gewählt aus der Gruppe Stärke und/oder dessen Derivate sowie der Gumen enthält. Diese können die Emulsion vorteilhaft stabilisieren. Die besten Stabilisierungsergebnisse werden dabei erzielt, wenn die Stärken (insbesondere Tapiokastärke) bei 50-100 °C in der Emulsion aufgequollen werden.

Als Stärken werden dabei erfindungsgemäß bevorzugt Distärkephosphat und Tapiokastärke eingesetzt, wobei die vorteilhafte Einsatzkonzentration für Distärkephosphat von 0,1 bis 10 Gewichts-% und für Tapiokastärke von 0,1 bis 20 Gewichts-%, jeweils bezogen auf das Gesamtgewicht der Zubereitung der zweiten Kammer, beträgt.

Zu den erfindungsgemäß bevorzugten Gumen zählt insbesondere Xanthan Gum, wobei die vorteilhafte Einsatzkonzentration für Xanthan Gum von 0,1 bis 1 Gewichts-%, bezogen auf das Gesamtgewicht der Zubereitung der zweiten Kammer, beträgt.

Auch ist es erfindungsgemäß von Vorteil, wenn die Zubereitung in der zweiten Kammer Glycerylstearat und/oder Cetearylalkohol enthält. Beide Stoffe stabilisieren die Zubereitung in der zweiten Kammer. Dabei ist es erfindungsgemäß bevorzugt, die Kombination aus Glycerylstearat und Cetearylalkohol einzusetzen.

Die vorteilhafte Einsatzkonzentration für Glycerylstearat beträgt von 0,1 bis 5 Gewichts-% und für Cetearylalkohol von 0,1 bis 5 Gewichts-%, jeweils bezogen auf das Gesamtgewicht der Zubereitung der zweiten Kammer.

Erfindungsgemäß vorteilhaft enthält die Zubereitung in der zweiten Kammer ein oder mehrere Parfümstoffe. Dabei ist es erfindungsgemäß bevorzugt, wenn die Parfümstoffe ausschließlich in der Zubereitung der zweiten Kammer enthalten sind. In diesem Falle kann der Einsatz von, beim Verbraucher, unerwünschten Lösungsvermittlern wie PEG-40 hydriertes Rizinusöl in der Gelphase vermieden werden.

Vorteilhafte Ausführungsformen der vorliegenden Erfindung sind dadurch gekennzeichnet, dass die Zubereitung in der zweiten Kammer einen oder mehrere Parfümstoffe gewählt aus der Gruppe der Verbindungen Verbindungen Limonen, Citral, Linalool, alpha-Isomethylionon, Geraniol, Citronellol, 2-lsobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C, Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Ethylenbrassylat, Evernia Furfuracea Extract, Evernia Prunastri Extract, Farnesol, Guajakholzöl, Geraniol, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Thymol, Tonkabohnenöl, Triethylcitrat und/oder Vanillin, enthält.

Außerdem ist es erfindungsgemäß von Vorteil, wenn die Zubereitung in der zweiten Kammer Polyethylengelycol-Stearatester enthält. Erfindungsgemäß bevorzugt ist dabei eine Gesamt-Einsatzkonzentration von 0,1 bis 7 Gewichts-%, bezogen auf das Gesamtgewicht der Zubereitung in der zweiten Kammer. Die erfindungsgemäß bevorzugten Polyethylengelycol-Stearatester sind dabei PEG-150 Distearate und PEG-40 Stearat.

Es ist vorteilhaft im Sinne der vorliegenden Erfindung, wenn mindestens eine der beiden Teilzubereitungen Glycerin enthält.

Es ist erfindungsgemäß vorteilhaft, wenn beide Teilzubereitungen frei sind von Propyl- und Butylparaben, 3-lod-2-propinylbutylcarbamat, Hydroxyisohexyl 3-Cyclohexene Carboxalhdehyde (Lyral), 2,6-Dihydroxy-4-methylbenzaldehyd (Atranol), 3-Chlor-2,6-dihydroxy-4-methylbenzaldehyd (Chloratranol), 3-(4-Methylbenzyliden)-campher, 2-Hydroxy-4-methoxybenzophenon (Oxybenzon), 4-Methoxyzimtsäure-2-ethylhexylester (Ethylhexyl Methoxycinnamate) und Octocrylen. Erfindungsgemäß bevorzugt wird darüber hinaus auch auf Methyl- und Ethylparaben verzichtet.

Erfindungsgemäß vorteilhafte Ausführungsformen sind dadurch gekennzeichnet, dass das Entnahmeverhältnis (Volumenverhältnis) bei der Entnahme der Teilzubereitungen aus dem Vorratsgefäß von 1:1,25 bis 1,25:1 beträgt. Dabei ist ein Entnahmeverhältnis von 1:1 erfindungsgemäß bevorzugt. Dabei ist natürlich zu berücksichtigen, dass es bei der Entnahme einzelner Teilzubereitungen immer zu geringen Volumenschwankungen kommen kann.

Es ist erfindungsgemäß von Vorteil, wenn die Viskosität der beiden zwei unterschiedlichen kosmetischen Teilzubereitungen, die über eine gemeinsame Entnahmeöffnung entnommen werden, nach dem Vermischen der beiden Teilzubereitungen von 1000 bis 15000 mPaS beträgt. Erfindungsgemäß bevorzugt ist dabei eine Viskosität von 5000 bis 10000 mPas.

Die erfindungsgemäß vorteilhafte Viskosität für das erfindungsgemäße Gel in der ersten Kammer beträgt von 7000 bis 15000 mPas. Die erfindungsgemäß vorteilhafte Viskosität für die erfindungsgemäße Zubereitung in der zweiten Kammer beträgt von 5000 bis 13000 mPas.

Die Viskositäten werden erfindungsgemäß gemessen mit dem Gerät Rheomat R 123 mit der Spindel Messkörper 1 und der konstanten Drehzahl von 62,5 pro min bei der Temperatur 25°C.

Darüber hinaus können die beiden Teilzubereitungen die üblichen für kosmetische Gele und Emulsionen bekannten Inhaltsstoffe enthalten, beispielsweise Wasser, EDTA, Öle (z.B. Dimethicon), Fette, Wachse, Alkohole wie Isopropylalkohol, t- Butylalkohol, BHT etc.

Es können für das Kosmetikum die handelsüblichen Zwei-Kammerpackmittel mit gemeinsamer Entnahmeöffnung verwendet werden, soweit sie für Zubereitungen mit unterschiedlicher Viskosität geeignet sind, beispielsweise der Dual Airless Dispenser von HANA Co., Ltd, Bestellnummer 67865 DUAL.PUMP.DISP.SILVER.MATT und 67866 DUAL.CHAMBER.CONT AINER.WHITE/SILVER.

### Beispiel

Das nachfolgende Beispiel soll die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Zubereitung erste Kammer: wässrig-alkoholisches Gel A** | |
|---|---|
| **INCI** | **m** [%] |
| Alcohol Denat. + Aqua | 12,00 |
| Glycerin + Aqua | 5,00 |
| Sodium Hyaluronate + Aqua | 1,00 |
| Aqua + Sodium Hydroxide | 0,30 |
| Carbomer | 1,00 |
| Phenoxyethanol | 0,60 |
| Aqua | 80,10 |
| **Total** | **100,00** |

| **Zubereitung erste Kammer: wässrig-alkoholisches Gel B** | |
|---|---|
| **INCI** | **m** [%] |
| Alcohol Denat. + Aqua | 10,00 |
| Glycerin + Aqua | 5,00 |
| Sodium Hyaluronate + Aqua | 0,50 |
| Aqua + Sodium Hydroxide | 0,30 |
| Carbomer | 1,00 |
| Phenoxyethanol | 0,60 |
| Aqua | 82,60 |
| **Total** | **100,00** |

| **Zubereitung erste Kammer: wässrig-alkoholisches Gel C** | |
|---|---|
| **INCI** | **m** [%] |
| Alcohol Denat. + Aqua | 8,00 |
| Glycerin + Aqua | 7,00 |
| Sodium Hyaluronate + Aqua | 0,10 |
| Aqua + Sodium Hydroxide | 0,30 |
| Carbomer | 1,00 |
| Phenoxyethanol | 0,60 |
| Aqua | 83,00 |
| **Total** | **100,00** |

| **Zubereitung zweite Kammer: O/W Emulsion A** | |
|---|---|
| **INCI** | **m** [%] |
| Cetearyl Alcohol | 3,00 |
| PEG-150 Distearate | 1,00 |
| PEG-40 Stearate | 2,00 |
| Aqua + Trisodium EDTA | 1,00 |
| Gluconolactone | 2,00 |
| Glycolic Acid + Aqua | 10,00 |
| Glycerin + Aqua | 5,00 |
| Glycine Soja Germ Extract | 2,00 |
| Sodium Hyaluronate + Aqua | 0,10 |
| Lactic Acid + Aqua | 2,00 |
| Glyceryl Stearate | 3,00 |
| Aqua + Sodium Hydroxide | 5,57 |
| Panthenol + Aqua | 0,30 |
| Parfum | 0,40 |
| Xanthan Gum | 0,50 |
| Dimethicone | 5,00 |
| Distarch Phosphate | 2,00 |
| Tapioca Starch + Aqua | 2,00 |
| Aqua | 53,13 |
| Total | **100,00** |

| Zubereitung zweite Kammer: O/W Emulsion B | |
|---|---|
| **INCI** | **m** [%] |
| Cetearyl Alcohol | 3,00 |
| PEG-150 Distearate | 1,00 |
| PEG-40 Stearate | 2,00 |
| Aqua + Trisodium EDTA | 1,00 |
| Gluconolactone | 3,50 |
| Glycolic Acid + Aqua | 8,00 |
| Glycerin + Aqua | 4,00 |
| Glycine Soja Germ Extract | 0,10 |
| Sodium Hyaluronate + Aqua | 0,80 |
| Lactic Acid + Aqua | 2,00 |
| Glyceryl Stearate | 3,00 |
| Aqua + Sodium Hydroxide | 5,57 |
| Panthenol + Aqua | 3,00 |
| Parfum | 0,40 |
| Xanthan Gum | 0,50 |
| Dimethicone | 5,00 |
| Distarch Phosphate | 2,00 |
| Tapioca Starch + Aqua | 5,00 |
| Aqua | 50,13 |
| Total | **100,00** |

| Zubereitung zweite Kammer: O/W Emulsion C | |
|---|---|
| **INCI** | **m** [%] |
| Cetearyl Alcohol | 3,00 |
| PEG-150 Distearate | 1,00 |
| PEG-40 Stearate | 2,00 |
| Aqua + Trisodium EDTA | 1,00 |
| Gluconolactone | 1,00 |
| Glycolic Acid + Aqua | 10,00 |
| Glycerin + Aqua | 7,00 |
| Glycine Soja Germ Extract | 1,10 |
| Sodium Hyaluronate + Aqua | 0,50 |
| Lactic Acid + Aqua | 1,00 |
| Glyceryl Stearate | 3,00 |
| Aqua + Sodium Hydroxide | 3,86 |
| Panthenol + Aqua | 0,80 |
| Parfum | 0,40 |
| Xanthan Gum | 0,50 |
| Dimethicone | 5,00 |
| Distarch Phosphate | 2,00 |
| Tapioca Starch + Aqua | 4,00 |
| Aqua | 52,84 |
| Total | **100,00** |

## Patentansprüche

1. Kosmetikum gebildet aus einem Vorratsbehältnis mit zwei separaten Vorratskammern, enthaltend zwei unterschiedliche kosmetische Teilzubereitungen, die über eine gemeinsame Entnahmeöffnung gleichzeitig entnommen werden, wobei die erste Kammer ein wässrig oder wässrig-alkoholisches Gel und die zweite Kammer eine Säure-haltige Zubereitung mit einem pH-Wert unter pH 4 enthält.

2. Kosmetikum nach Anspruch 1, **dadurch gekennzeichnet, dass** das Gel in der ersten Kammer durch Polyacrylat (Carbomer) verdickt ist.

3. Kosmetikum nach einem der vorhergehenden Ansprüche, **dadurch gekennzeichnet, dass** das Gel in der ersten Kammer Polyacrylate in einer Konzentration von 0,1 bis 1,5 Gewichts-%, bezogen auf das Gesamtgewicht des Gels in der ersten Kammer, enthält.

4. Kosmetikum nach einem der vorhergehenden Ansprüche, **dadurch gekennzeichnet, dass** das Gel in der ersten Kammer ein oder mehrere Kosmetische Wirkstoffe gewählt aus der Gruppe der Verbindungen D-Biotin, Coenzym Q10, alpha-Glucosylrutin, Carnitin, Carnosin, natürliche und/oder synthetische Isoflavonoide, Flavonoide, Kreatin, Kreatinin, Taurin, ß-Alanin, Panthenol, Magnolol, Honokiol, Polydocanol, Glycerylglycose, Tocopherolacetat, Natriumhyaluronat, Harnstoff und/oder Licochalcon A enthält.

5. Kosmetikum nach einem der vorhergehenden Ansprüche, **dadurch gekennzeichnet, dass** das Gel in der ersten Kammer Phenoxyethanol, Ethylhexylglycerin und/oder 4-Hydroxyacetophenon enthält.

6. Kosmetikum nach einem der vorhergehenden Ansprüche, **dadurch gekennzeichnet, dass** das Gel in der ersten Kammer ein wässrig-alkoholisches Gel darstellt mit einem Ethanol-Gehalt von mindestens 4 Gew.-% Ethanol, bezogen auf das Gesamtgewicht des Gels in der ersten Kammer.

7. Kosmetikum nach einem der vorhergehenden Ansprüche, **dadurch gekennzeichnet, dass** die Zubereitung in der zweiten Kammer ein oder mehrere Säuren, gewählt aus der Gruppe der Verbindungen Gluconsäure, Milchsäure, Ascorbinsäure, Zitronensäure, Salizylsäure, Glykolsäure enthält.

8. Kosmetikum nach einem der vorhergehenden Ansprüche, **dadurch gekennzeichnet, dass** die die Zubereitung in der zweiten Kammer in Form einer Öl-in-Wasser-Emulsion (O/W-Emulsion) vorliegt.

9. Kosmetikum nach einem der vorhergehenden Ansprüche, **dadurch gekennzeichnet, dass** die Zubereitung in der zweiten Kammer ein oder mehrere Verbindungen gewählt aus der Gruppe Stärke und/oder dessen Derivate sowie der Gumen enthält.

10. Kosmetikum nach einem der vorhergehenden Ansprüche, **dadurch gekennzeichnet, dass** die Zubereitung in der zweiten Kammer Glycerylstearat und/oder Cetearylalkohol enthält.

11. Kosmetikum nach einem der vorhergehenden Ansprüche, **dadurch gekennzeichnet, dass** die Zubereitung in der zweiten Kammer einen oder mehrere Parfümstoffe gewählt aus der Gruppe der Verbindungen Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C, Butylphenylmethyl-propionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Ethylenbrassylat, Evernia Furfuracea Extract, Evernia Prunastri Extract, Farnesol, Guajakholzöl, Geraniol, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Thymol, Tonkabohnenöl, Triethylcitrat und/oder Vanillin, enthält.

12. Kosmetikum nach einem der vorhergehenden Ansprüche, **dadurch gekennzeichnet, dass** die Zubereitung in der zweiten Kammer Polyethylengelycol-Stearatester enthält.

13. Kosmetikum nach einem der vorhergehenden Ansprüche, **dadurch gekennzeichnet, dass** der pH-Wert des wässrig oder wässrig-alkoholisches Gels in der ersten Kammer größer als pH 4,9 ist.

14. Kosmetikum nach einem der vorhergehenden Ansprüche, **dadurch gekennzeichnet, dass** beide Teilzubereitungen frei sind von Propyl- und Butylparaben, 3-lod-2-propinylbutylcarbamat, Hydroxyisohexyl 3-Cyclohexene Carboxalhdehyde (Lyral), 2,6-Dihydroxy-4-methylbenzaldehyd (Atranol), 3-Chlor-2,6-dihydroxy-4-methylbenzaldehyd (Chloratranol), 3-(4-Methylbenzyliden)-campher, 2-Hydroxy-4-methoxybenzophenon (Oxybenzon), 4-Methoxyzimtsäure-2-ethylhexylester (Ethylhexyl Methoxycinnamate) und Octocrylen.

15. Kosmetikum nach einem der vorhergehenden Ansprüche, **dadurch gekennzeichnet, dass** das Entnahmeverhältnis (Volumenverhältnis) bei der Entnahme der Teilzubereitungen aus dem Vorratsgefäß von 1:1,25 bis 1,25:1 beträgt.

16. Kosmetikum nach einem der vorhergehenden Ansprüche, **dadurch gekennzeichnet**, das die Viskosität der beiden zwei unterschiedliche kosmetische Teilzubereitungen, die über eine gemeinsame Entnahmeöffnung entnommen werden, nach dem Vermischen der beiden Teilzubereitungen von 1000 bis 15000 mPaS beträgt, gemessen mit dem Gerät Rheomat R 123 mit der Spindel Messkörper 1 der konstanten Drehzahl von 62,5 pro min bei der Temperatur 25°C.

## Claims

1. Cosmetic formed from a storage container having two separate storage chambers, comprising two different cosmetic part preparations, which are withdrawn simultaneously through a common discharge opening, wherein the first chamber comprises an aqueous or aqueous alcoholic gel and the second chamber comprises an acid-containing preparation having a pH less then pH 4.

2. Cosmetic according to Claim 1, **characterized in that** the gel in the first chamber is thickened by polyacrylate (carbomer).

3. Cosmetic according to either of the preceding claims, **characterized in that** the gel in the first chamber comprises polyacrylates at a concentration of 0.1% to 1.5% by weight, based on the total weight of the gel in the first chamber.

4. Cosmetic according to any of the preceding claims, **characterized in that** the gel in the first chamber comprises one or more cosmetic active ingredients selected from the group of compounds comprising D-biotin, coenzyme Q10, alpha-glucosylrutin, carnitine, carnosine, natural and/or synthetic isoflavonoids, flavonoids, creatine, creatinine, taurine, β-alanine, panthenol, magnolol, honokiol, polidocanol, glycerylglucose, tocopherol acetate, sodium hyaluronate, urea and/or licochalcone A.

5. Cosmetic according to any of the preceding claims, **characterized in that** the gel in the first chamber comprises phenoxyethanol, ethylhexylglycerin and/or 4-hydroxyacetophenone.

6. Cosmetic according to any of the preceding claims, **characterized in that** the gel in the first chamber is an aqueous alcoholic gel having an ethanol content of at least 4% by weight ethanol, based on the total weight of the gel in the first chamber.

7. Cosmetic according to any of the preceding claims, **characterized in that** the preparation in the second chamber comprises one or more acids selected from the group of compounds comprising gluconic acid, lactic acid, ascorbic acid, citric acid, salicylic acid, glycolic acid.

8. Cosmetic according to any of the preceding claims, **characterized in that** the preparation in the second chamber is in the form of an oil-in-water emulsion (O/W emulsion).

9. Cosmetic according to any of the preceding claims, **characterized in that** the preparation in the second chamber comprises one or more compounds selected from the group of starch and/or derivatives thereof and also gums.

10. Cosmetic according to any of the preceding claims, **characterized in that** the preparation in the second chamber comprises glyceryl stearate and/or cetearyl alcohol.

11. Cosmetic according to any of the preceding claims, **characterized in that** the preparation in the second chamber comprises one or more perfumes selected from the group of compounds comprising limonene, citral, linalool, alpha-isomethyl ionone, geraniol, citronellol, 2-isobutyl-4-hydroxy-4- methyltetrahydropyran, 2-tert-pentylcyclohexyl acetate, 3-methyl-5-phenyl-1- pentanol, 7-acetyl-1,1,3,4,4,6-hexamethyltetralin, adipic acid diesters, alpha-amylcinnamaldehyde, alpha-methyl ionone, amyl C, butylphenylmethylpropionalcinnamal, amyl salicylate, amylcinnamyl alcohol, anise alcohol, benzoin, benzyl alcohol, benzyl benzoate, benzyl cinnamate, benzyl salicylate, bergamot oil, bitter orange oil, butylphenyl methylpropional, cardamom oil, cedrol, cinnamal, cinnamyl alcohol, citronellyl methylcrotonate, lemon oil, coumarin, diethyl succinate, ethyl linalool, eugenol, ethylene brassylate, Evernia furfuracea extract, Evernia prunastri extract, farnesol, guaiac wood oil, geraniol, hexylcinnamal, hexyl salicylate, hydroxycitronellal, lavender oil, limonene oil, linayl acetate, mandarin oil, menthyl PCA, methylheptenone, nutmeg oil, rosemary oil, sweet orange oil, terpineol, thymol, tonka bean oil, triethyl citrate and/or vanillin.

12. Cosmetic according to any of the preceding claims, **characterized in that** the preparation in the second chamber comprises polyethylene glycol stearate esters.

13. Cosmetic according to any of the preceding claims, **characterized in that** the pH of the aqueous or aqueous alcoholic gel in the first chamber is greater than pH 4.9.

14. Cosmetic according to any of the preceding claims, **characterized in that** both part preparations are free from propyl paraben and butyl paraben, 3-iodo-2-propynyl butylcarbamate, hydroxyisohexyl 3-cyclohexene carboxaldehyde (Lyral), 2,6-dihydroxy-4-methylbenzaldehyde (Atranol), 3-chloro-2,6-dihydroxy-4-methylbenzaldehyde (Chloratranol), 3-(4-methylbenzylidene)camphor, 2-hydroxy-4-methoxybenzophenone (Oxybenzone), 2-ethylhexyl 4-methoxycinnamate (Ethylhexyl Methoxycinnamate) and octocrylene.

15. Cosmetic according to any of the preceding claims, **characterized in that** the discharge ratio (volume ratio) during discharge of the part preparations from the storage vessel is from 1:1.25to 1.25:1.

16. Cosmetic according to any of the preceding claims, **characterized in that** the viscosity of the two different cosmetic part preparations, which are withdrawn through a common discharge opening, after mixing of the two part preparations, is from 1000 to 15 000 mPaS, measured with a Rheomat R 123 instrument using the spindle measuring bob 1 at a constant speed of 62.5 revolutions per minute at a temperature of 25°C.

## Revendications

1. Produit cosmétique formé à partir d'un récipient de réserve comportant deux compartiments de réserve séparés, contenant deux préparations cosmétiques partielles différentes, qui peuvent être prélevées simultanément par le biais d'une ouverture de prélèvement commune, le premier compartiment contenant un gel aqueux ou aqueux-alcoolique et le deuxième compartiment contenant une préparation contenant un acide dotée d'une valeur de pH inférieure à 4.

2. Produit cosmétique selon la revendication 1, **caractérisé en ce que** le gel est épaissi dans le premier compartiment par un polyacrylate (carbomère).

3. Produit cosmétique selon l'une quelconque des revendications précédentes, **caractérisé en ce que** le gel dans le premier compartiment contient des polyacrylates en une concentration de 0,1 à 1,5 % en poids, par rapport au poids total du gel dans le premier compartiment.

4. Produit cosmétique selon l'une quelconque des revendications précédentes, **caractérisé en ce que** le gel dans le premier compartiment contient un ou plusieurs principes actifs cosmétiques choisis dans le groupe des composés D-biotine, coenzyme Q10, alpha-glucosylrutine, carnitine, carnosine, isoflavonoïdes naturels et/ou synthétiques, flavonoïdes, créatine, créatinine, taurine, β-alanine, panthénol, magnolol, honokiol, polydocanol, glycérylglucose, acétate de tocophérol, hyaluronate de sodium, urée et/ou licochalcone A.

5. Produit cosmétique selon l'une quelconque des revendications précédentes, **caractérisé en ce que** le gel dans le premier compartiment contient du phénoxyéthanol, de l'éthylhexylglycérine et/ou de la 4-hydroxyacétophénone.

6. Produit cosmétique selon l'une quelconque des revendications précédentes, **caractérisé en ce que** le gel dans le premier compartiment est un gel aqueux-alcoolique doté d'une teneur en éthanol d'au moins 4 % en poids d'éthanol, par rapport au poids total du gel dans le premier compartiment.

7. Produit cosmétique selon l'une quelconque des revendications précédentes, **caractérisé en ce que** la préparation dans le deuxième compartiment contient un ou plusieurs acides, choisis dans le groupe des composés acide gluconique, acide lactique, acide ascorbique, acide citrique, acide salicylique, acide glycolique.

8. Produit cosmétique selon l'une quelconque des revendications précédentes, **caractérisé en ce que** la préparation dans le deuxième compartiment est présente sous forme d'une émulsion huile-dans-eau (émulsion H/E).

9. Produit cosmétique selon l'une quelconque des revendications précédentes, **caractérisé en ce que** la préparation dans le deuxième compartiment contient un ou plusieurs composés choisis dans le groupe des amidons et/ou de leurs dérivés ainsi que des gommes.

10. Produit cosmétique selon l'une quelconque des revendications précédentes, **caractérisé en ce que** la préparation dans le deuxième compartiment contient du stéarate de glycéryle et/ou de l'alcool cétéarylique.

11. Produit cosmétique selon l'une quelconque des revendications précédentes, **caractérisé en ce que** la préparation dans le deuxième compartiment contient une ou plusieurs matières parfumées choisies dans le groupe des composés limonène, citral, linalool, alpha-isométhylionone, géraniol, citronellol, 2-isobutyl-4-hydroxy-4-méthyltétrahydropyrane, acétate de 2-tert-pentylcyclohexyle, 3-méthyl-5-phényl-1-pentanol, 7-acétyl-1,1,3,4,4,6-hexaméthyltétraline, diester de l'acide adipique, alpha-amylcinnamaldéhyde, alpha-méthylionone, amyl C butylphénylméthylpropionalcinnamal, salicylate d'amyle, alcool amylcinnamylique, alcool anisique, benzoïne, alcool benzylique, benzoate de benzyle, cinnamate de benzyle, salicylate de benzyle, huile de bergamote, huile d'orange amère, butylphénylméthylpropional, huile de cardamome, cédrol, cinnamal, alcool cinnamylique, crotonate de citronellylméthyle, huile de citron, coumarine, succinate de diéthyle, éthyllinalool, eugénol, brassylate d'éthylène, extrait d'Evernia Furfuracea, extrait d'Evernia Prunastri, farnésol, huile de bois de gaïac, géraniol, hexylcinnamal, salicylate d'hexyle, hydroxycitronellal, huile de lavande, huile de limonène, acétate de linayle, huile de mandarine, menthyl-PCA, méthylhepténone, huile de noix de muscade, huile de romarin, huile d'orange douce, terpinéol, thymol, huile de fèves de tonka, citrate de triéthyle et/ou vanilline.

12. Produit cosmétique selon l'une quelconque des revendications précédentes, **caractérisé en ce que** la préparation dans le deuxième compartiment contient un ester de stéarate d'un polyéthylène glycol.

13. Produit cosmétique selon l'une quelconque des revendications précédentes, **caractérisé en ce que** la valeur de pH du gel aqueux ou aqueux-alcoolique dans le premier compartiment est supérieure au pH 4,9.

14. Produit cosmétique selon l'une quelconque des revendications précédentes, **caractérisé en ce que** les deux préparations partielles sont exemptes de propylparabène et butylparabène, de 3-iodo-2-propinylbutylcarbamate, d'hydroxyisohexyl-3-cyclohexènecarboxaldéhyde (lyral), de 2,6-dihydroxy-4-méthylbenzaldéhyde (atranol), de 3-chloro-2,6-dihydroxy-4-méthylbenzaldéhyde (chloratranol), de 3-(4-méthylbenzylidène)-camphre, de 2-hydroxy-4-méthoxybenzophénone (Oxybenzon), d'ester de 2-éthylhexyle d'acide 4-méthoxycinnamique (Ethylhexyl Methoxycinnamate) et d'octocrylène.

15. Produit cosmétique selon l'une quelconque des revendications précédentes, **caractérisé en ce que** le rapport de prélèvement (rapport en volume) lors du prélèvement des préparations partielles du récipient de réserve est de 1 : 1,25 à 1,25 : 1.

16. Produit cosmétique selon l'une quelconque des revendications précédentes, **caractérisé en ce que** la viscosité des deux préparations cosmétiques partielles différentes qui sont prélevées par le biais d'une ouverture de prélèvement commune, après le mélange des deux préparations partielles, est de 1 000 à 15 000 mPa.s, mesurée avec l'appareil Rheomat R 123 avec le corps de mesure de broche 1 à la vitesse de rotation constante de 62,5 tpm à la température de 25 °C.
